(19) Europäisches Patentamt

European Patent Office

Office européen des brevets

(11) Veröffentlichungsnummer: **0 315 857 B1**

(12) **EUROPÄISCHE PATENTSCHRIFT**

(45) Veröffentlichungstag der Patentschrift: **10.06.92**

(21) Anmeldenummer: **88118091.3**

(22) Anmeldetag: **31.10.88**

(51) Int. Cl.⁵: **C07C  53/128**, C07C 51/235

(54) Verfahren zur Herstellung von alpha-verzweigten C12-C40-Fettsäuren.

(30) Priorität: **10.11.87 DE 3738198**

(43) Veröffentlichungstag der Anmeldung:
**17.05.89 Patentblatt  89/20**

(45) Bekanntmachung des Hinweises auf die
Patenterteilung:
**10.06.92 Patentblatt  92/24**

(84) Benannte Vertragsstaaten:
**DE FR GB IT**

(56) Entgegenhaltungen:
**EP-A- 0 112 261**
**US-A- 2 353 159**

**CHEMICAL ABSTRACTS, Band 19, Nr. 19, 7.**
**Mai 1979, Seite 557, Nr. 151586e, Columbus,**
**Ohio, US; & JP-A-78 135 919**

(73) Patentinhaber: **Henkel Kommanditgesellschaft**
**auf Aktien**
**Postfach 1100 Henkelstrasse 67**
**W-4000 Düsseldorf 1(DE)**

(72) Erfinder: **Tran-Vinh, Loc, Dr.**
**1300 Santee Drive 1**
**San Jose, CA 95122(US)**
Erfinder: **Carduck, Franz-Josef, Dr.**
**Landstrasse 18**
**W-5657 Haan(DE)**
Erfinder: **Göbel, Gerd, Dr.**
**Lindenstrasse 10**
**W-4006 Erkrath(DE)**

**Beschreibung**

Alpha-verzweigte $C_{12}$-$C_{40}$-Fettsäuren, z.B. die bei Umgebungstemperatur flüssige 2-Hexyldecansäure (Isopalmitinsäure), sind infolge ihres gesättigten Charakters wesentlich farb- und oxidationsstabiler als vergleichbare ungesättigte Fettsäuren, z.B. Ölsäure. Sie sind daher wertvolle Zwischenprodukte für die Herstellung u.a. von Emulgatoren, kosmetischen Produkten, Wasch-, Textil-, Gummiund Kunststoffhilfsmitteln sowie Schmiermitteln.

Alpha-verzweigte $C_{12}$-$C_{40}$-Fettsäuren werden üblicherweise nach dem Alkalischmelsverfahren, z.B. gemäß DE-OS 23 20 461, durch Oxidation der entsprechenden alpha-verzweigten $C_{12}$-$C_{40}$-Alkanole in einer Natrium- und/oder Kaliumhydroxidschmelze hergestellt. Nachteile dieses Verfahrens sind die hohe Betriebstemperatur, die Korrosivität der entstehenden Seifen und die erforderliche destillative Abtrennung des Hauptprodukts von den Nebenprodukten, welche je nach Betriebsbedingungen ca. 4 bis 20 Gew.-% ausmachen können.

Ein attraktives, alternatives Verfahren für die Herstellung von alpha-verzweigten Fettsäuren ist die Luftoxidation der entsprechenden Alkohole in einem heterogen-katalytischen Prozeß, wobei insbesondere Platin und Palladium als Katalysatoren verwendet werden können DE 3135946A1, DE 2936123A1, DE 34465561A1. Die Oxidation von alpha-verzweigten $C_{12}$-$C_{40}$-Alkanolen mit derartigen Edelmetallkatalysatoren verläuft jedoch wenig selektiv und erfordert den Einsatz von relativ großen Mengen an Edelmetallen. Die Wiedergewinnung des Katalysators, der dabei auftretende Katalysatorverlust sowie die thermische Produktreinigung stellen die Wirtschaftlichkeit eines derartigen Verfahrens in Frage.

Aus **Jpn.Kokai Tokkyo Koho 78,135,919** zitiert nach **Chem.Abstr. Vol. 90, 151586e** ist ferner ein Verfahren zur Herstellung von 2-Ethylhexansäure bekannt, bei dem man eine wäßrige Emulsion des entsprechenden Alkohols in Gegenwart eines Pd/C-Katalysators und eines anionischen Tensids bei 60 bis 70°C mit Sauerstoff oxidiert.

Demgemäß ist die Erfindung auf ein Verfahren der eingangs genannten Art gerichtet, das mit relativ geringen Katalysatorkonzentrationen und damit kostengünstig arbeitet und eine hohe Produktselektivität aufweist.

Diese Aufgabe wird erfindungsgemäß dadurch gelöst, daß man alpha-verzweigte $C_{12}$-$C_{40}$-Alkanole in flüssiger insbesondere wäßriger Phase mit Sauerstoff bzw. Sauerstoff enthaltenden Gasen in Gegenwart von wirksamen Mengen eines Palladium-Katalysators und Blei(II)-Verbindungen als Aktivator umsetzt sowie die $C_{12}$-$C_{40}$-Fettsäuren aus den erhaltenen Alkalisalzen derselben in üblicher Weise freisetzt und isoliert.

Bei dem Verfahren der Erfindung werden somit Platin-Katalysatoren durch das billigere Palladium ersetzt; der Zusatz an Blei(II)-Verbindungen erhöht sowohl den Umsatz der Reaktion als auch die Selektivität; weiterhin wird die für einen bestimmten Umsatz erforderliche Palladiumkonzentration im Reaktionsgemisch durch den Zusatz der Aktivatoren gesenkt. Die Reaktion kommt nach Aufnahme der stöchiometrisch erforderlichen Sauerstoffmenge von selbst zum Stillstand, muß also nicht mehr rechtzeitig - zur Vermeidung von Überoxidationen - abgebrochen werden. Dies war unter den alkalischen Reaktionsbedingungen gemäß dem Stand der Technik bisher nicht der Fall.

Es konnte nicht erwartet werden, daß die erfindungsgemäß eingesetzte Kombination von Palladium und Blei(II)-Verbindungen bei dem Verfahren der Erfindung zu einer Steigerung von Ausbeute und Selektivität führt. Zwar beschreibt die DE-OS 31 35 946 ein Verfahren zur Herstellung von Ethercarbonsäuren durch Oxidation der entsprechenden Alkohole mit Sauerstoff bzw. Sauerstoff enthaltenden Gasen in Gegenwart von Palladium-Katalysatoren und Metallverbindungen als Aktivatoren, gemäß Beispiel 15 dieser Veröffentlichung wird jedoch gerade mit der Kombination Palladium/Blei(II)-Verbindungen ein völlig unbrauchbares Ergebnis erzielt.

Gemäß einer vorteilhaften Ausführungsform der Erfindung stellt man die alpha-verzweigten $C_{12}$-$C_{40}$-Fettsäuren her, indem man die entsprechenden $C_{12}$-$C_{40}$-Alkanole der allgemeinen Formel

$$R\text{-}CH_2\text{-}CH_2\text{-}CHR\text{-}CH_2OH$$

in der die Gruppen R jeweils gleiche $C_4$-$C_{18}$-Alkylgruppen bedeuten, oxidiert. Hierfür besonders geeignete $C_{12}$-$C_{40}$-Alkanole sind die sogenannten Guerbetalkanole, deren Herstellungsweise in der genannten DE-OS 23 20 461 beschrieben ist und die als Dimerisierungsprodukte von unverzweigten Alkoholen wie n-Octylalkohol, n-Nonylalkohol, n-Decylalkohol, n-Undecylalkohol, n-Dodecylalkohol, n-Tridecylalkohol, n-Tetradecylalkohol, n-Pentadecylalkohol und Eicosanol betrachtet werden können.

Bei dem Verfahren der Erfindung erfolgt die Oxidation des alpha-verzweigten $C_{12}$-$C_{40}$-Alkanols in Gegenwart von Alkalien, vorzugsweise von Hydroxiden und/oder Carbonaten des Natriums und/oder Kaliums. Im allgemeinen werden ca. 1 bis 1,5 Mol Alkali pro Mol 2-Hexyldecanol eingesetzt. Die Alkalien können zu Reaktionsbeginn auf einmal zugefügt werden; es ist aber auch möglich, nach der Einstellung eines alkalischen Ausgangs-pH-Wertes das Alkali im Verlauf der Reaktion in Portionen oder kontinuierlich in dem Maß zuzugeben, wie es durch die entstehende $C_{12}$-$C_{40}$-Fettsäure verbraucht wird.

Das als Katalysator zu verwendende Palladium kann den Reaktionskomponenten in elementarer Form, gegebenenfalls in Kombination mit anderen Platinmetallen oder in Form von Verbindungen, z.B. Palladiumoxid, -chlorid oder -acetat, zugegeben werden. Das Edelmetall kann auch auf festen Trägern aufgebracht werden. Besonders bevorzugt sind Palladium/Aktivkohle-Katalysatoren. Der Edelmetallgehalt der Trägerkatalysatoren kann in weiten Bereichen schwanken und beträgt vorzugsweise 0,1 bis 20 Gew.-%. Die Palladium-Katalysatoren werden dem Reaktionsgemisch in einer Menge entsprechend 0,01 bis 20 g, insbesondere 0,1 bis 10 g Palladium pro Mol eingesetztem $C_{12}$-$C_{40}$-Alkanol zugesetzt.

Erfindungsgemäß kommen als Aktivatoren Blei-(II)-Verbindungen zum Einsatz, die aus der von Oxiden, Hydroxiden, Salzen anorganischer oder organischer Säuren wie Chloriden, Nitraten oder Acetaten, Salzen von Übergangsmetallen enthaltenden Sauerstoffsäuren wie Vanadaten, Komplexverbindungen und metallorganischen Verbindungen gebildeten Gruppe ausgewählt sind. Die Aktivatoren können im Reaktionsgemisch jeweils löslich, teilweise löslich oder unlöslich sein. Man kann die Aktivatoren schon bei der Herstellung des Palladium-Katalysators zugeben oder den Palladium-Katalysator mit dem Aktivator imprägnieren. Die Aktivatoren können dem Reaktionsgemisch aber auch von den Katalysatoren getrennt zugesetzt werden. Weiterhin kann der Aktivator auch als Trägermaterial für das Palladium dienen.

Bevorzugt setzt man die Blei(II)-Verbindungen in einer Menge von $1.10^{-5}$ bis $1.10^{-1}$, insbesondere $2.10^{-5}$ bis $2.10^{-2}$ Mol pro Mol eingesetztem $C_{12}$-$C_{40}$-Alkanol zu.

Gemäß einer weiteren vorteilhaften Ausführungsform der Erfindung führt man die Oxidation bei Temperaturen zwischen $0\,°C$ und dem Siedepunkt des Reaktionsgemisches, vorzugsweise zwischen 40 und $100\,°C$, durch. Der einzustellende Betriebsdruck ist vor allem abhängig vom Partialdruck des Sauerstoffs des verwendeten Gases und liegt vorzugsweise zwischen 0,5 bis 40 bar.

Der in Wasser unlösliche $C_{12}$-$C_{40}$-Alkanol läßt sich in wäßrig-alkalischen Medien mit alpha-verzweigten $C_{12}$-$C_{40}$-Fettsäuren emulgieren. Gemäß einer weiteren vorteilhaften Ausführungsform der Erfindung setzt man demgemäß dem Reaktionsgemisch vor der Umsetzung 0,001 bis 1 Mol, insbesondere 0,1 bis 0,4 Mol alpha-verzweigte $C_{12}$-$C_{40}$-Fettsäure pro Mol $C_{12}$-$C_{40}$-Alkanol zu. Man arbeitet dann unter den Bedingungen einer micellaren Katalyse, die unter Umständen den Reaktionsverlauf günstig beeinflußt.

Das Verfahren der Erfindung kann in allen Apparaturen, die sich für die Durchführung von Flüssigphasenreaktionen mit oder ohne Anwendung von Überdruck eignen, durchgeführt werden, z.B. in einem Rührkessel, in einer Blasensäule mit suspendiertem Katalysator oder in einem Rieselbettreaktor mit Katalysatorpellets.

Nach der Abtrennung des Katalysators erhält man als Endprodukte der Oxidationsreaktion eine wäßrige Lösung von Alkalisalzen der $C_{12}$-$C_{40}$-Fettsäure, aus der das gewünschte Produkt durch Ansäuern mit Mineralsäuren, beispielsweise mit Schwefelsäure, Salzsäure oder Phosphorsäure freigesetzt und durch Phasentrennung isoliert wird. Die erhaltene $C_{12}$-$C_{40}$-Fettsäure kann destillativ gereinigt werden.

Das Verfahren der Erfindung wird im folgenden anhand bevorzugter Ausführungsbeispiele näher erläutert.

Beispiel 1.

In einem mit Drehrührer, Innenthermometer und Gaszuleitung versehenen, über einen Heizmantel temperierbaren 3 1-Autoklaven aus Hastelloy B wurden 500 g destilliertes Wasser, 15 g Aktivkohle mit 5 Gew.-% Palladiumgehalt, 1 g Pb-$(NO_3)_2$, 9,9 g Ätznatron, 50 g technisches 2-Hexyldecanol (ca. 85 %) und 15 g Isopalmitinsäure (95 %-ig) eingebracht; der Isopalmitinsäurezusatz erfolgte zur Emulgierung des wasserunlöslichen 2-Hexyldecanols.

Der Rührer wurde angestellt und das Reaktionsgemisch auf $90\,°C$ gebracht. Bei dieser Temperatur wurde Syntheseluft (mit 2l Vol.-% $O_2$) bei 20 bar mit einem Volumenstrom von 30 Nl Luft pro Stunde durch den Reaktor geleitet. Der Reaktionsverlauf wurde mit einem Sauerstoffanalysator am Reaktorausgang verfolgt. Der Versuch wurde nach 5 Stunden Reaktionszeit abgebrochen.

Nach Abfiltrieren des Katalysators wurde das Filtrat mit verdünnter Schwefelsäure angesäuert und zur Bestimmung der Ausbeute mehrfach mit Ether ausgeschüttelt. Von den vereinigten Etherextrakten wurde der Ether - nach Trocknen über Natriumsulfat - abgezogen. Die so aufbereitete Probe wurde mit Hilfe der Hochdruckflüssigkeitschromatografie quantitativ untersucht. Der Umsatz betrug 83 Gew.-%, die erreichte Selektivität 100 Gew.-%.

Beispiel 2.

Es wurde wie in Beispiel 1 verfahren, jedoch unter Zusatz von 1 g PbO als Aktivator.

Der erzielte Umsatz betrug 81 Gew.-%, die Selektivität 100 Gew.-%.

Vergleichsbeispiel.

Es wurde wie in Beispiel 1 verfahren, jedoch unter alleinigem Zusatz von 15 g Aktivkohle mit 5 Gew.-% Palladiumgehalt ohne Aktivatorzusatz. Der Umsatz betrug 52 Gew.-%, die Selektivität 23 Gew.-%, wobei im HPLC-Chromatogramm 4 nicht identifizierte Verbindungen als Nebenprodukte beobachtet wurden.

Das Vergleichsbeispiel zeigt, daß sich mit der erfindungsgemäßen Palladium/Aktivator-Kombination sowohl Umsatz als auch Selektivität stark verbessern lassen.

## Patentansprüche

1. Verfahren zur Herstellung von alpha-verzweigten $C_{12}$-$C_{40}$-Fettsäuren durch Oxidation der entsprechenden alpha-verzweigten $C_{12}$-$C_{40}$-Alkanole in Gegenwart von Alkalien, dadurch gekennzeichnet, daß man die $C_{12}$-$C_{40}$-Alkanole in flüssiger, insbesondere wäßriger Phase mit Sauerstoff bzw. Sauerstoff enthaltenden Gasen in Gegenwart von wirksamen Mengen eines Palladium-Katalysators und Blei(II)-Verbindungen als Aktivator umsetzt sowie die $C_{12}$-$C_{40}$-Fettsäuren aus den erhaltenen Alkalisalzen derselben in üblicher Weise freisetzt und isoliert.

2. Verfahren nach Anspruch 1, dadurch gekennzeichnet, daß man alpha-verzweigte $C_{12}$-$C_{40}$-Alkanole der allgemeinen Formel

R-CH$_2$-CH$_2$-CHR-CH$_2$OH

in der die Gruppen R jeweils gleiche $C_4$-$C_{18}$-Alkylgruppen bedeuten, oxidiert.

3. Verfahren nach Anspruch 1 oder 2, dadurch gekennzeichnet, daß man als Katalysator auf einem festen Träger aufgebrachtes Palladium verwendet.

4. Verfahren nach einem der Ansprüche 1 bis 3, dadurch gekennzeichnet, daß der feste Träger 0,1 bis 20 Gew.-% Palladium enthält.

5. Verfahren nach einem der Ansprüche 1 bis 4, dadurch gekennzeichnet, daß man einen Palladium/Aktivkohle-Katalysator verwendet.

6. Verfahren nach einem der Ansprüche 1 bis 5, dadurch gekennzeichnet, daß man den Palladium-Katalysator in einer Menge entsprechend 0,01 bis 20 g, insbesondere 0,1 bis 10 g Palladium pro Mol eingesetztem $C_{12}$-$C_{40}$-Alkanol verwendet.

7. Verfahren nach einem der Ansprüche 1 bis 6, dadurch gekennzeichnet, daß man als Aktivator eine Blei(II)-Verbindung verwendet, die aus der von Oxiden, Hydroxiden, Salzen anorganischer oder organischer Säuren, Salzen von Übergangsmetallen enthaltenden Sauerstoffsäuren, Komplexverbindungen und metallorganischen Verbindungen gebildeten Gruppe ausgewählt ist.

8. Verfahren nach einem der Ansprüche 1 bis 7, dadurch gekennzeichnet, daß man die Blei(II)-Verbindungen in einer Menge von $1.10^{-5}$ bis $1.10^{-1}$, insbesondere $2.10^{-5}$ bis $2.10^{-2}$ Mol pro Mol eingesetztem $C_{12}$-$C_{40}$-Alkanol verwendet.

9. Verfahren nach einem der Ansprüche 1 bis 8, dadurch gekennzeichnet, daß man die Oxidation in wäßriger Phase in Gegenwart von etwa 1 bis 1,5 Mol Alkali pro Mol $C_{12}$-$C_{40}$-Alkanol durchführt.

10. Verfahren nach einem der Ansprüche 1 bis 9, dadurch gekennzeichnet, daß man Alkalien aus der von Hydroxiden und Carbonaten des Natriums und Kaliums gebildeten Gruppe verwendet.

11. Verfahren nach einem der Ansprüche 1 bis 10, dadurch gekennzeichnet, daß man die Oxidation bei Temperaturen zwischen 0°C und dem Siedepunkt des Reaktionsgemisches durchführt.

12. Verfahren nach einem der Ansprüche 1 bis 11, dadurch gekennzeichnet, daß man die Oxidation bei Temperaturen zwischen 40 und 100°C durchführt.

13. Verfahren nach einem der Ansprüche 1 bis 12, dadurch gekennzeichnet, daß man die Oxidation bei einem Druck zwischen 0,5 und 40 bar durchführt.

14. Verfahren nach einem der Ansprüche 1 bis 13, dadurch gekennzeichnet, daß man dem Reaktionsgemisch vor Beginn der Umsetzung 0,001 bis 1 Mol, insbesondere 0,1 bis 0,4 Mol $C_{12}$-$C_{40}$-Fettsäure pro Mol $C_{12}$-$C_{40}$-Alkanol zusetzt.

## Claims

1. A process for the production of alpha-branched $C_{12-40}$ fatty acids by oxidation of the corresponding alpha-branched $C_{12-40}$ alkanols in the presence of alkalis, characterized in that the $C_{12-40}$ alkanols are reacted with oxygen or oxygen-containing gases in liquid, particularly aqueous, phase in the presence of effective quantities of a palladium catalyst and lead(II) compounds as activator and the $C_{12-40}$ fatty acids are released from the resulting alkali metal salts thereof and isolated by known methods.

2. A process as claimed in claim 1, characterized in that alpha-branched $C_{12-40}$ alkanols corresponding to the following general formula

$R$-$CH_2$-$CH_2$-$CHR$-$CH_2OH$

in which the groups R are the same and represent $C_{4-18}$ alkyl groups, are oxidized.

3. A process as claimed in claim 1 or 2, characterized in that palladium applied to a solid support is used as the catalyst.

4. A process as claimed in any of claims 1 to 3, characterized in that the solid support contains 0.1 to 20% by weight palladium.

5. A process as claimed in any of claims 1 to 4, characterized in that a palladium/active carbon catalyst is used.

6. A process as claimed in any of claims 1 to 5, characterized in that the palladium catalyst is used in a quantity corresponding to 0.01 to 20 g and more especially to 0.1 to 10 g palladium per mol $C_{12-40}$ alkanol used.

7. A process as claimed in any of claims 1 to 6, characterized in that the activator used is a lead(II) compound selected from the group consisting of oxides, hydroxides, salts of inorganic or organic acids, oxy acids containing salts of transition metals, complex compounds and organometallic compounds.

8. A process as claimed in any of claims 1 to 7, characterized in that the lead(II) compounds are used in a quantity of $1.10^{-5}$ to $1.10^{-1}$ mol and more especially in a quantity of $2.10^{-5}$ to $2.10^{-2}$ mol per mol $C_{12-40}$ alkanol used.

9. A process as claimed in any of claims 1 to 8, characterized in that the oxidation is carried out in the aqueous phase in the presence of about 1 to 1.5 mol alkali per mol $C_{12-40}$ alkanol.

10. A process as claimed in any of claims 1 to 9 characterized in that alkalis from the group consisting of hydroxides and carbonates of sodium and potassium are used.

11. A process as claimed in any of claims 1 to 10, characterized in that the oxidation is carried out at temperatures between 0°C and the boiling point of the reaction mixture.

12. A process as claimed in any of claims 1 to 11, characterized in that the oxidation is carried out at temperatures between 40 and 100°C.

13. A process as claimed in any of claims 1 to 12, characterized in that the oxidation is carried out under a pressure of 0.5 to 40 bar.

14. A process as claimed in any of claims 1 to 13, characterized in that 0.001 to 1 mol and more especially 0.1 to 0.4 mol $C_{12-40}$ fatty acid per mol $C_{12-40}$ alkanol is added to the reaction mixture before the beginning of the reaction.

**Revendications**

1. Procédé de production d'acides gras $\alpha$-ramifiés, en $C_{12}$ à $C_{40}$ par oxydation des alcanols $\alpha$-ramifiés, en $C_{12}$ - $C_{40}$ correspondants en présence d'alcalis, caractérisé en ce que l'on fait réagir les alcanols en $C_{12}$-$C_{40}$ en phase liquide, en particulier aqueuse, avec de l'oxygène ou de gaz contenant de l'oxygène, en présence de quantités efficaces d'un catalyseur au palladium et de composés du Plomb (II) comme activateur et en ce qu'on libère les acides gras en $C_{12}$-$C_{40}$ à partir des sels alcalins de ceux-ci obtenus, d'une manière usuelle et qu'on les isole.

2. Procédé selon la revendication 1, caractérisé en ce que l'on oxyde les alcanols en $C_{12}$- $C_{40}$ $\alpha$-ramifiés en de formule générale :

$R$-$CH_2$-$CH_2$-$CHR$-$CH_2OH$

dans laquelle les radicaux R signifient respectivement les mêmes groupes alcoyle en $C_4$-$C_{18}$.

3. Procédé selon la revendication 1 ou 2, caractérisé en ce que l'on utilise comme catalyseur, du palladium appliqué sur un support solide.

4. Procédé selon une des revendications 1 à 3, caractérisé en ce que le support solide renferme de 0,1 à 20 % en poids de palladium.

5. Procédé selon l'une des revendications 1 à 4, caractérisé en ce que l'on utilise un catalyseur charbon actif/palladium.

6. Procédé selon l'une des revendications 1 à 5, caractérisé en ce que l'on utilise le catalyseur au palladium en quantité correspondant à 0,01 jusqu'à 20 g - en particulier de 0,1 à 10 g - de palladium par mol d'alcanol en $C_{12}$-$C_{40}$ mis en jeu.

7. Procédé selon l'une des revendications 1 à 6, caractérisé en ce que comme activateur, on utilise un composé de plomb (II) qui est choisi dans le groupe formé par des oxydes, hydroxydes, sels d'acides minéraux ou organiques, sels d'acides oxygénés renfermant des métaux de transition, composés complexes, et composés organo-métalliques.

8. Procédé selon l'une des revendications 1 à 7, caractérisé en ce que l'on utilise les composés de plomb (II) en quantité allant de $1 \cdot 10^{-5}$ à $1 \cdot 10\text{-}1$ - en particulier de $2\ 10^{-5}$ à $2 \cdot 10^{-2}$ mol - par mol d'alcanol en $C_{12}$-$C_{40}$ mis en oeuvre.

9. Procédé selon l'une des revendications 1 à 8, caractérisé en ce que l'on effectue l'oxydation en phase aqueuse en présence d'environ 1 à 1,5 mol d'alcali par mol d'alcanol en $C_{12}$-$C_{40}$.

10. Procédé selon l'une des revendications 1 à 9, caractérisé en ce que l'on utilise des alcalis choisis dans le groupe formé des hydroxydes et des carbonates de sodium et de potassium.

11. Procédé selon l'une des revendications 1 à 10, caractérisé en ce que l'on effectue l'oxydation à des températures comprises entre 0° et le point d'ébullition du mélange réactionnel.

12. Procédé selon l'une des revendications 1 à 11, caractérisé en ce que l'on effectue l'oxydation à des températures comprises entre 40 et 100°C.

13. Procédé selon l'une des revendications 1 à 12, caractérisé en ce que l'on effectue l'oxydation à une pression comprise entre 0,5 et 40 bar.

14. Procédé selon l'une des revendications 1 à 13, caractérisé en ce que l'on additionne au mélange réactionnel avant le début de la réaction, 0,001 à 1 mol - en particulier 0,1 à 0,4 mol - d'acide gras en $C_{12}$-$C_{40}$, par mole d'alcanol en $C_{12}$ à $C_{40}$.